Europäisches Patentamt

European Patent Office

Office européen des brevets

(19)

(11) Publication number: **0 040 914**
**B1**

(12) **EUROPEAN PATENT SPECIFICATION**

(45) Date of publication of patent specification: **10.08.83**

(21) Application number: **81301861.1**

(22) Date of filing: **28.04.81**

(51) Int. Cl.³: **C 07 C 1/20, C 10 G 3/00, B 01 J 29/38**

(54) Process for the catalytic conversion of methanol into hydrocarbons boiling within the motor fuel range.

(30) Priority: **22.05.80 US 152458**

(43) Date of publication of application:
**02.12.81 Bulletin 81/48**

(45) Publication of the grant of the patent:
**10.08.83 Bulletin 83/32**

(84) Designated Contracting States:
**BE DE FR GB IT NL**

(56) References cited:
**EP - A - 0 009 917**
**GB - A - 2 033 358**
**US - A - 4 118 339**
**US - A - 4 148 835**
**US - A - 4 174 272**

(73) Proprietor: **MOBIL OIL CORPORATION**
**150 East 42nd Street**
**New York New York 10017 (US)**

(72) Inventor: **Gross, Benjamin**
**1693 Blue Jay Lane**
**Cherry Hill New Jersey (US)**
Inventor: **Kuo, James Cheng-wu**
**1017 Heartwood Drive**
**Cherry Hill New Jersey (US)**
Inventor: **Voltz, Sterling Ernst**
**6 E Glen Circle**
**Media Pennsylvania (US)**

(74) Representative: **West, Alan Harry**
**Mobil Court 3 Clements Inn**
**London WC2A 2EB (GB)**

Courier Press, Leamington Spa, England

Process for the catalytic conversion of methanol into hydrocarbons boiling within the motor fuel range

This invention relates generally to the conversion of methanol into gasoline and, more particularly, to the control of carbon monoxide emissions during regeneration of the spent methanol conversion catalyst.

The conversion of methanol into gasoline is an important area of technology which has the potential of becoming even more important as the supply of crude oil is diminished and/or crude oil is increased in price. Particularly advantageous catalysts which are utilized for the conversion of methanol into gasoline are crystalline aluminosilicate zeolites of which ZSM—5 is the most preferred. There are many patents which describe the conversion of methanol into gasoline over such zeolites, including United States Patents 3,931,349; 3,969,426; 3,899,544; 3,894,107; 3,907,914; and 3,894,102. Also United States Patent 4,148,835 describes the conversion of methanol into hydrocarbons, mainly light olefins, over a catalyst comprising ZSM—5 together with zinc, palladium and magnesium oxide.

It is known in the art that the catalytic conversion of methanol into gasoline over zeolites such as ZSM—5 results in the deposition of carbonaceous material, generally referred to as coke, on the catalyst resulting in a decline of catalyst activity which must be compensated for by reactivation or regeneration of the catalyst by burning off the coke at elevated temperatures in a regenerator. Among the products resulting from the combustion of this coke are carbon monoxide and carbon dioxide. Recent environmental regulations have seriously limited the amount of carbon monoxide which can be discharged to the atmosphere and there is therefore a need for a process wherein carbon monoxide can be combusted in the regenerator so as to minimize atmospheric pollution.

Although there is much art involving the combustion of carbon monoxide in a regenerator, this art is really concerned with the catalytic cracking of gas oil to produce gasoline and not the catalytic conversion of methanol into gasoline. Art dealing with the control of carbon monoxide from a catalytic cracking process is represented by United States Patents 4,072,600; 4,088,568; and 4,093,535. Also, United States Patent 4,174,272 describes the addition to the inventory of cracking catalyst circulating in a cracking unit, of a decomposable compound of a platinum group metal so as to obtain an inventory containing 0.1 to 100 ppm of CO-oxidation promoting metal.

The present invention is based on the observation that trace amounts of metals of Periods 5 and 6 of Group VIII of the Periodic Table and rhenium permit effective control of carbon monoxide from the effluent gas of the regenerator, while still enabling excellent gasoline yield and quality to be maintained.

The present invention therefore provides a process for the catalytic conversion of methanol into hydrocarbons boiling within the motor fuel range, which comprises the steps of contacting methanol with a catalyst comprising porous, acidic, solid particles comprising a crystalline aluminosilicate zeolite having a pore diameter greater than 5 Angstroms, a silica-to-alumina ratio of at least 12 and a constraint index of from 1 to 12, in a reaction vessel at a temperature between 260 and 538°C, under a pressure between 200 and 3550 kPa and at a liquid hourly space velocity between 0.3 and 10 in the absence of added hydrogen; transferring the used catalyst bearing deactivating solid carbonaceous contaminant from the reaction vessel to a regeneration vessel; contacting the used catalyst in the regeneration vessel with air to oxidize the carbonaceous contaminant into carbon monoxide and carbon dioxide; and transferring the regenerated catalyst from the regeneration vessel to the reaction vessel to catalyze further conversion of methanol into hydrocarbons; characterized in that the catalyst also comprises particles of an oxidation catalyst comprising at least one metal selected from platinum, palladium, iridium, osmium, rhodium, ruthenium and rhenium in an amount of less than 100 ppm based on the total catalyst, to promote oxidation of carbon monoxide in the regeneration vessel but not substantially to cause dealkylation of aromatic hydrocarbon conversion products in the reaction vessel.

Thus, platinum, palladium, rhodium, ruthenium, iridium, osmium and rhenium and mixtures thereof have been found to possess an extraordinarily high activity in connection with CO oxidation activity and yet, when used in such small amounts, not to have an adverse effect on gasoline quality and yield in a methanol conversion process. It is surprising that these very active metals can be used in such minute amounts that they will retain their oxidation activity and yet their hydrogenation/dehydrogenation activity can be suppressed so as not seriously to affect the methanol conversion reaction. The use of these metals therefore provides flexibility with regard to controlling CO emissions to the atmosphere which is mandated by various environmental regulations. As pointed out above, the broad concept of adding these metals to the catalyst inventory of a cracking unit is known and is disclosed in United States Patents 4,072,600; 4,088,568; 4,093,535; and 4,174,272. However, these patents are concerned with the catalytic cracking of gas oil to produce gasoline rather than the conversion of methanol into gasoline. Quite obviously, different reactions are involved.

The process of this invention involves the addition of at least one metal of Periods 5 and 6 of Group VIII of the Periodic Table and rhenium, to the circulating catalyst inventory of a methanol conversion unit in an amount such that the conversion of carbon monoxide to carbon dioxide in the regenerator will be considerably enhanced and yet the methanol conversion reaction will be substantially unaffected.

It is known in the art to convert methanol into gasoline utilizing crystalline aluminosilicate zeolites such as ZSM—5. In addition, some of the patents mentioned above contain teachings that the crystalline aluminosilicate zeolites can have a hydrogenation/dehydrogenation function associated therewith. In this connection, specific mention is made of United States Patent 3,969,426, column 3, lines 18—17; and United States Patent 3,899,544, column 3, lines 51—68 and claim 8. Quite clearly, if a ZSM—5 type zeolite having a dehydrogenation component is regenerated in the presence of an appropriate amount of air, the catalyst will inherently oxidize the CO to $CO_2$ even though such a reaction is not expressly disclosed in those patents.

However, the process of this invention is not concerned merely with controlling the carbon monoxide emission resulting from the regeneration of spent catalyst, since there is another facet which is critical in the process of this invention and that is to accomplish the control of carbon monoxide without substantially affecting the quality or quantity of the gasoline which is produced.

In this connection, although the above patents involving the conversion of methanol into gasoline with catalysts such as ZSM—5 do, indeed, teach the inclusion of metals having a hydrogenation/dehydrogenation function, nevertheless, there is a difference in the gasoline which is produced depending upon whether or not a hydrogenation component is used and whether or not the conversion is carried out in the presence of added hydrogen. In general, the use of catalysts such as ZSM—5 without an added hydrogenation component and in the absence of added hydrogen results in the production of gasoline in very high yields with small quantities of light gas and practically no production of hydrogen. The gasoline contains large concentrations of paraffins (which are mostly isoparaffins) and aromatics and has a very high octane level. The gasoline has good stability at reasonable additive levels.

Thus, the process of this invention involves not only maintaining all the advantages of converting methanol into gasoline with catalysts such as ZSM—5 that are inherent to such a system when it is carried out in the absence of added hydrogen and the absence of dehydrogenation/hydrogenation components, but also the benefit with regard to CO oxidation activity that inherently results from using a hydrogenation component with ZSM—5. This is accomplished by carefully controlling the amount of metals which are added to the ZSM—5 type zeolite such that substantially no hydrogenation/dehydrogenation activity is present in the reactor because of the low level of metals which are used, yet enabling these metals still to provide sufficient oxidation activity in the regenerator to catalyze the combustion of CO to $CO_2$. The amount of added metal is therefore limited to a level not to exceed 100 ppm and more preferably of from 2 to 10 ppm, based on the total catalyst inventory. It has been observed that unless the metal concentration in the circulating catalyst inventory of a conversion unit is limited to no more than 100 ppm, unacceptable gasoline make will occur. Metal concentrations of from 1 to 50 ppm are also desirable, particularly at high regenerator temperatures. It is, indeed, surprising that catalysts containing these low levels would possess sufficient oxidation activity in the regenerator effectively to control carbon monoxide emission and yet have their dehydrogenation/hydrogenation activity sufficiently minimized so as not detrimentally to affect the methanol conversion reaction.

It is to be understood that the specific CO oxidation components need not be present in the form of the metal *per se* but may be present as compounds thereof, for example as oxides or sulfides.

Two major variants for converting methanol are fluid processes and moving bed processes. In both of these processes, the methanol feed and the catalyst are passed through a "reactor"; are disengaged; the catalyst is regenerated with cocurrent and/or countercurrent air; and the regenerated reflexively heated catalyst is recontacted with more feed to start the cycle again. These two processes differ substantially in the size of the catalyst particles utilized and also in the engineering of materials contact and transfer which is at least partially a function of the catalyst size.

In fluid processes, the catalyst is a fine powder of 10 to 200 microns, preferably about 70 micron, size. This fine powder is generally propelled upwardly through a riser reaction zone suspended in and thoroughly mixed with methanol feed. The coked catalyst particles are separated from the conversion products and, after being purged, are transferred into the regenerator where coke is burned to reactivate the catalyst. Regenerated catalyst generally flows downward from the regenerator to the base of the riser.

In a typical example of a moving bed process, the catalyst is in the shape of beads or pellets having an average particle size of 0.4 to 6.5 mm, preferably about 0.32 mm. Active, hot catalyst beads progress downwardly cocurrent with a charge stock through a reaction zone. In this zone, feed is converted while coke is deposited on the catalyst. At the lower end of the reaction zone, the products are separated from the coked catalyst and recovered. The coked catalyst is then passed downwardly to a regeneration zone, into which air is fed such that part of the air passes upwardly countercurrent to the coked catalyst and part of the air passes downwardly cocurrent with partially regenerated catalyst. Two flue gases comprising carbon oxides are produced. Regenerated catalyst is disengaged from the flue gas and is then lifted, pneumatically or mechanically, to the top of the reaction zone.

It is to be understood that the particular method of adding the desired metal to the circulating catalyst inventory is not critical and, in fact, can be performed in a number of different ways. The metals of Periods 5 and 6 of Group VIII and rhenium may be a component of all of the catalyst particles or of only some of the catalyst particles. In terms of its concentration in the entire system, however, it must be present in a large enough proportion to be able to effect the reaction of carbon monoxide with oxygen to carbon dioxide (provided that the conditions in the regenerator are otherwise sufficient to support this combustion, i.e. sufficiently high temperature and sufficient air). Nevertheless, it must not be present in a proportion so large that it substantially adversely affects the operation of the conversion side of the process. In this regard, it is important to note that in some instances it may be desirable to cause substantially all of the carbon burned in the regenerator to be oxidized all the way to carbon dioxide. In other instances, it may be desirable to cause only part of the carbon to be oxidized all the way to carbon dioxide and permit some substantial amount to be only partially oxidized to carbon monoxide. Because of the inherent advantages stemming from the use of the process of this invention, it is possible to increase the regenerator temperature by burning some of the carbon monoxide therein, burning the rest outside the regenerator, for example, in a steam-generating CO-boiler. Quite clearly, the preferred features of this invention reside in burning all of the carbon monoxide within the regenerator since this obviates the need for a CO-incinerator or boiler which may be necessary in order to meet the various environmental regulations concerning CO emission to the atmosphere.

A convenient method of adding the desired metal to the circulating catalyst inventory is to form a mixture of the metal or metals with an inorganic oxide such as alumina, and to add the alumina containing metal either to the conversion catalyst *per se* or to any component thereof prior to its introduction into the conversion unit. Another method of adding the oxidation catalyst to the conversion unit is to introduce the alumina containing metal directly into a unit which already contains conversion catalyst, such introduction being accomplished either by adding it to the regenerator or to the unit. It is to be understood, however, that other inorganic oxides besides alumina can be used. Other suitable materials include for example, silica-alumina, silica-magnesia and clays.

The metal component may also be incorporated into the catalyst by impregnation, by ion exchange or by other means by contacting either the catalyst or a component thereof with a solution of a compound of the metal in an appropriate amount necessary to provide the desired concentration of the metal. The metal component may be incorporated either in any step during preparation of the catalyst or after the otherwise finished catalyst has been prepared. A preferred manner of incorporation is to ion-exchange a crystalline aluminosilicate and then composite the ion-exchanged product with a porous matrix. Also useful is the ion-exchanging or impregnation of siliceous solids or clays. Suitable metal compounds include the metal halides, preferably chlorides, nitrates, ammine halides, oxides, sulfates, phosphates and other watersoluble inorganic salts; and also the metal salts of carboxylic acids having from 1 to 5 carbon atoms, and alcoholates. Specific examples include palladium chloride, chloroplatinic acid, ruthenium penta-ammine chloride, osmium chloride, perrhenic acid, dioxobis (ethylenediamine) rhenium (V) chloride and rhodium chloride. Alternatively, a methanol-soluble or methanol-dispersable compound of the metal may be added in a suitable amount to the methanol feed for incorporation into the catalyst as the charge is converted. Such compounds include metal diketonates, carbonyls, metallocenes, olefin complexes of 2 to 20 carbons, acetylene complexes, alkyl or aryl phosphine complexes and carboxylates of 1 to 20 carbons. Specific examples of these are platinum acetylacetonate, tris (acetylacetonato) rhodium (III), triiodoiridium (III) tricarbonyl, $\pi$ - cyclopentadienylrhenium (I) tricarbonyl, ruthenocene, $\pi$-cyclopentadienyl osmium (I) dicarbonyl dimer, dichloro (ethylene) palladium (II) dimer, ($\pi$-cyclopentadienyl) (ethylene) rhodium (I), diphenyl acetylenebis (triphenyl-phosphino) platinum (O), bromomethylbis (triethylphosphino) palladium (II), tetrakis (triphenylphosphino) palladium (O), chloro-carbonylbis(triphenylphosphino) iridium (I), palladium acetate, and palladium naphthenate.

Regardless of the method of incorporating the metal component in the catalyst, improved results are obtained.

The methanol conversion catalyst used in the method described herein comprises a crystalline aluminosilicate zeolite having a pore diameter greater than 5 Angstroms, a silica-to-alumina ratio of at least 12 and a constraint index of from 1 to 12.

A pore diameter greater than about 5 Angstroms results from pore windows of about a size such as would be provided by 10- membered rings of oxygen atoms. It is to be understood, of course, that these rings are those formed by the regular disposition of the tetrahedra making up the anionic framework of the crystalline aluminosilicate, the oxygen atoms themselves being bonded to the silicon or aluminum atoms at the centers of the tetrahedra. This important characteristic of the crystal structure of such zeolites provides constrained access to, and egress from the intracrystalline free space.

The silica-to-alumina ratio may be determined by conventional analysis. This ratio is meant to represent, as closely as possible, the ratio in the rigid anionic framework of the zeolite crystal and to exclude aluminum in any binder present or in cationic or other form within the channels. Although zeolites with a silica-to-alumina ratio of at least 12 are useful, it is preferred to use zeolites having higher ratios of at least about 30.

The significance and method of determination of the constraint index are fully described in, for example United States Patent 3,905,915.

The zeolites defined herein are exemplified by ZSM—5, ZSM—11, ZSM—12, ZSM—35 and ZSM—38 and other similar materials. ZSM—5 is described in U.S. Patent 3,702,886; ZSM—11 is described in U.S. Patent 3,709,979; ZSM—12 is described in U.S. Patent 3,832,449; ZSM—35 is described in U.S. Patent 4,016,245; and ZSM—38 is described in U.S. Patent 4,046,859.

These specific zeolites, when prepared in the presence of organic cations, are catalytically inactive, possibly because the intracrystalline free space is occupied by organic cations from the forming solution. They may be activated by heating in an inert atmosphere at 538°C for one hour, for example, followed by base exchange with ammonium salts followed by calcination at 538°C in air. The presence of organic cations in the forming solution may not be absolutely essential to the formation of this type of zeolite; however, the presence of these cations does appear to favor the formation of this type of catalyst by base exchange with ammonium salts followed by calcination in air at about 538°C for from about 15 minutes to about 24 hours.

Natural zeolites may sometimes be converted to this type of zeolite catalyst by various activation procedures and other treatments such as base exchange, steaming, alumina extraction and calcination, in combinations. Natural minerals which may be so treated include ferrierite, brewsterite, stilbite, dachiardite, epistilbite, heulandite, and clinoptilolite. However, the preferred crystalline aluminosilicates are ZSM—5, ZSM—11, ZSM—12, ZSM—38 and ZSM—35, ZSM—5 being particularly preferred.

In a preferred aspect of this invention, the zeolites are those having a crystal framework density, in the dry hydrogen form, of not substantially below about 1.6 grams per cubic centimeter. The significance and determination of crystal framework densities are described in, for example, U.S. Patent 4,137,148.

The crystal size of the synthesized zeolite is generally within the approximate range of 0.01 to 40 microns.

The zeolites used in the process of this invention can have the original cations associated therewith replaced by a wide variety of other cations according to techniques well known in the art. Typical replacing cations include hydrogen, ammonium and metal cations including mixtures thereof. Of the replacing metallic cations, particular preference is given to cations of metals such as rare earth metals, manganese and calcium.

Typical ion exchange techniques involve contact of the particular zeolite with a salt of the desired replacing cation or cations. Although a wide variety of salts can be employed, particular preference is given to chlorides, nitrates and sulfates.

Representative ion exchange techniques are disclosed in a wide variety of patents, including United States Patents 3,140,249; 3,140,251; and 3,140,253.

Following contact with the salt solution of the desired replacing cation, the zeolites are then preferably washed with water and dried at a temperature ranging from 65°C to 316°C and thereafter calcined in air or an inert gas at temperatures ranging from 260 to 816°C for periods of time ranging from 1 to 48 hours or more.

Prior to use, the zeolites should be dehydrated at least partially. This can be achieved by heating to a temperature in the range of 200 to 600°C in an atmosphere, such as air or nitrogen, and at atmospheric or subatmospheric pressures for between 1 and 48 hours. Dehydration can also be performed at lower temperatures merely by using a vacuum, but a longer time is required to obtain a sufficient amount of dehydration.

Operating conditions for methanol conversion include temperatures between 260 and 538°C, a pressure between 200 and 3550 kPa but preferably between 270 and 2500 kPa. The liquid hourly space velocity is generally between 0.3 and 10, preferably between 0.5 and 4.

The zeolites utilized in the process of this invention are preferably incorporated or otherwise admixed with a matrix. Matrix materials are well known in the art and include inorganic oxides such as clay, silica, alumina and silica-alumina. The matrix which is used is preferably non-catalytic, alumina being particularly preferred.

**Claims**

1. A process for the catalytic conversion of methanol into hydrocarbons boiling within the motor fuel range, which comprises the steps of:

contacting methanol with a catalyst comprising porous, acidic, solid particles comprising a crystalline aluminosilicate zeolite having a pore diameter greater than 5 Angstroms, a silica-to-alumina ratio of at least 12 and a constraint index of from 1 to 12, in a reaction vessel at a temperature between 260 and 538°C, under a pressure between 200 and 3550 kPa and at a liquid hourly space velocity between 0.3 and 10 in the absence of added hydrogen;

transferring the used catalyst bearing deactivating solid carbonaceous contaminant from the reaction vessel to a regeneration vessel;

contacting the used catalyst in the regeneration vessel with air to oxidize the carbonaceous contaminant into carbon monoxide and carbon dioxide; and

transferring the regenerated catalyst from the regeneration vessel to the reaction vessel to catalyze further conversion of methanol into hydrocarbons; characterized in that the catalyst also comprises particles of an oxidation catalyst comprising at least one metal selected from platinum, palladium, iridium, osmium, rhodium, ruthenium and rhenium in an amount of less than 100 ppm based on the total catalyst, to promote oxidation of carbon monoxide in the regeneration vessel but not substantially to cause dealkylation of aromatic hydrocarbon conversion products in the reaction vessel.

2. A process according to claim 1, wherein the catalyst includes from 1 to 50 ppm of said metal.

3. A process according to claim 1, wherein the catalyst includes from 2 to 10 ppm of said metal.

4. A process according to any one of claims 1 to 3, wherein the oxidation catalyst comprises platinum.

5. A process according to any one of claims 1 to 3, wherein the oxidation catalyst comprises palladium.

6. A process according to any one of claims 1 to 5, wherein the crystalline aluminosilicate zeolite is ZSM—5.

7. A process according to any one of claims 1 to 5, wherein the crystalline aluminosilicate zeolite is ZSM—11.

## Revendications

1. Procédé pour la conversion catalytique du méthanol en hydrocarbures bouillant dans la plage des combustibles pour moteur, qui comprend les stades de:

mise en contact du méthanol avec un catalyseur comprenant des particules poreuses, acides et solides, constitué d'un aluminosilicate cristallin zéolitique ayant un diamètre des pores supérieur à 5 Å, un rapport de la silice à l'alumine d'au moins 12 et un indice de contrainte de 1 à 12, dans un récipient réactionnel, à une température entre 260 et 538°C, sous une pression entre 200 et 3 550 kPa, et avec une vitesse spatiale liquide horaire entre 0,3 et 10, en l'absence d'hydrogène ajouté;

le transfert du catalyseur usé portant un contaminant carboné solide désactivant du récipient réactionnel à un récipient de régénération;

la mise en contact du catalyseur usé dans le récipient de régénération avec de l'air pour oxyder le contaminant carboné en monoxyde de carbone et en dioxyde de carbone; et

le transfert du catalyseur régénéré du récipient de régénération au récipient pour catalyser une nouvelle conversion du méthanol en hydrocarbures;

caractérisé en ce que le catalyseur comprend également des particules d'un catalyseur d'oxydation comprenant au moins un métal choisi parmi le platine, le palladium, l'iridium, l'osmium, le rhodium, le ruthénium et le rhénium, en une quantité inférieure à 100 ppm par rapport au catalyseur total, pour favoriser l'oxydation du monoxyde de carbone dans le récipient de régénération, mais sans provoquer pratiquement de désalkylation des produits de conversion hydrocarbonés aromatiques dans le récipient de réaction.

2. Un procédé selon la revendication 1, dans lequel le catalyseur comprend de 1 à 50 ppm dudit métal.

3. Un procédé selon la revendication 1, dans lequel le catalyseur comprend 2 à 10 ppm dudit métal.

4. Un procédé selon l'une quelconque des revendications 1 à 3, dans lequel le catalyseur d'oxydation comprend du platine.

5. Un procédé selon l'une quelconque des revendications 1 à 3, dans lequel le catalyseur d'oxydation comprend du palladium.

6. Un procédé selon l'une quelconque des revendications 1 à 5, dans lequel l'aluminosilicate cristallin zéolitique est la ZSM—5.

7. Un procédé selon l'une quelconque des revendications 1 à 5, dans lequel l'aluminosilicate cristallin zéolitique est la ZSM—11.

## Patentansprüche

1. Ein Verfahren zur katalytischen Umwandlung von Methanol in Kohlenwasserstoffe, die im Motorenkraftstoffbereich sieden, das die folgenden Schritte umfaßt:

Inkontaktbringen von Methanol mit einem Katalysator, der poröse, saure, feste Teilchen umfaßt, die einen kristallinen Alumosilikat-Zeolith umfassen, der einen Porendurchmesser von größer als 5 Å, ein Siliciumoxid-zu-Aluminiumoxid-Verhältnis von wenigstens 12 und einen Grenzwertindex (constraint index) von 1 bis 12 aufweist, in einem Reaktionsbehälter

bei einer Temperatur zwischen 260 und 538°C, bei einem Druck zwischen 200 und 3550 kPa und einer Raumgeschwindigkeit (liquid hourly space velocity) zwischen 0,3 und 10 in Abwesenheit von zugesetztem Wasserstoff;

Überführung des gebrauchten Katalysators, der eine desaktivierende feste, kohlenstoffhaltige Verschmutzung trägt, aus dem Reaktionsbehälter in einen Regenerationsbehälter;

Inkontaktbringen des gebrauchten Katalysators in dem Regenerationsbehälter mit Luft, um die kohlenstoffhaltige Verschmutzung in Kohlenmonoxid und Kohlendioxid zu oxidieren; und

Überführen des regenerierten Katalysators aus dem Regenerationsbehälter in den Reaktionsbehälter, um die weitere Umwandlung von Methanol in Kohlenwasserstoffe zu katalysieren;

dadurch gekennzeichnet, daß der Katalysator außerdem Teilchen eines Oxidationskatalysators umfaßt, der wenigstens ein Metall aus der Gruppe Platin, Palladium, Iridium, Osmium, Rhodium, Ruthenium und Rhenium in einer Menge von weniger als 100 ppm, bezogen auf den ganzen Katalysator, enthält, um die Oxidation von Kohlenmonoxid in dem Regenerations-Behälter zu fördern, jedoch keine wesentliche Entalkylierung von aromatischen Kohlenwasserstoff-Umwandlungsprodukten in dem Reaktionsbehälter zu bewirken.

2. Ein Verfahren nach Anspruch 1, bei dem der Katalysator von 1 bis 50 ppm des genannten Metalls umfaßt.

3. Ein Verfahren nach Anspruch 1, bei dem der Katalysator von 2 bis 10 ppm des genannten Metalls umfaßt.

4. Ein Verfahren nach einem der Ansprüche 1 bis 3, bei dem der Oxidationskatalysator Platin umfaßt.

5. Ein Verfahren nach einem der Ansprüche 1 bis 3, bei dem der Oxidationskatalysator Palladium umfaßt.

6. Ein Verfahren nach einem der Ansprüche 1 bis 5, bei dem der kristalline Alumosilikat-Zeolith ZSM—5 ist.

7. Verfahren nach einem der Ansprüche 1 bis 5, bei dem der kristalline Alumosilikat-Zeolith ZSM—11 ist.